(19) Europäisches Patentamt
European Patent Office
Office européen des brevets

(11) Publication number: **0 031 694**
**B1**

(12) **EUROPEAN PATENT SPECIFICATION**

(45) Date of publication of patent specification: 01.06.83

(51) Int. Cl.[3]: C 07 C 51/295, C 07 C 53/126

(21) Application number: 80304633.3

(22) Date of filing: 19.12.80

(54) **Production of carboxylic acid salts.**

(30) Priority: 26.12.79 US 106978

(43) Date of publication of application: 08.07.81 Bulletin 81/27

(45) Publication of the grant of the patent: 01.06.83 Bulletin 83/22

(84) Designated Contracting States: AT DE GB SE

(56) References cited:
GB - A - 660 838
GB - A - 785 296
US - A - 1 934 648
US - A - 2 926 182

ULLMANNS ENCYCLOPÄDIE DER TECHNISCHEN CHEMIE, 4th revised and enlarged edition, 1975, vol. 9 VERLAG CHEMIE, Weinheim, Bergstr. page 141

(73) Proprietor: STAUFFER CHEMICAL COMPANY
Westport Connecticut 06880 (US)

(72) Inventor: Magee, Walter Lee
Thaddeus Avenue
Danbury Connecticut 06810 (US)

(74) Representative: Smith, Sydney et al,
Elkington and Fife High Holborn House 52/54
High Holborn
London WC1V 6SH (GB)

EP 0 031 694 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Production of carboxylic acid salts

This invention relates to an improved process for producing carboxylic acid salts, in particular, an improved process which permits greater control of the process, thus producing reaction mixtures having a reduced tendency to foam and solidify.

Carboxylic acid salts and acids derived therefrom are useful for the preparation of high quality bar soaps and other soap products, the preparation of various types of esters for various purposes such as lubricants, hydraulic fluids, edible oils and fats and numerous applications as intermediates in the preparation of a wide variety of chemical compounds.

The preparation of carboxylic acid salts and of carboxylic acids via the caustic fusion reaction of alcohols, with or without a catalyst, is a process that has been known for many years (see, for example, Dumas and Stas, Ann., 35, 129—173, 1840 and U.S. Patent 2,384,817 to Chitwood). This process has of comparatively recent date become associated with the preparation of high purity salts and acids of a type capable of effective direct substitution for naturally derived salts and carboxylic acids in large use areas, such as the manufacture of high quality soap products where cost is an important factor, and the production of esters for use in hydraulic fluids and lubricants. One possible reason for this is that it was only recently that synthetic alcohols of high purity and low cost became available in quantities sufficient for consideration as raw materials for the production of such salts and acids. Until this stage of technology was reached, the usual derivation of alcohols was from the acid components of ester materials in natural source oils and fats such as coconut oil. Thus, prior large scale processing was directed to producing alcohols from acid structures, not vice versa, and this for the most part made natural source derived alcohols more costly than natural source salts and acids; thus, there was no prior reason for considering large scale production of natural source type salts and acids from alcohols by a caustic fusion process or by any process for that matter.

The caustic fusion reaction of alcohols, i.e. reacting an alcohol (ROH) with a caustic $M(OH)_n$ to produce carboxylic salts, as indicated previously, is well known in the art, and various improvements on the process and variations thereof are described in, for example, the following U.S. Patents: 2,384,817 to *Chitwood*; 2,614,122 to *Mikeska* (which describes preparing dodecanedioic acid by cleaving 12-hydroxy-stearic acid with an alkali metal hydroxide in the presence of high boiling saturated petroleum hydrocarbons solvent resulting in a viscous emulsified mass which is difficult to purify); 2,696,501 to *Stein*; 2,847,466 to *Steadmann et al.* (which describes a caustic fusion reaction in the presence of water); 3,121,728 to *Bartlett*; 3,227,737 to *Ashworth*; 3,365,476 to *Dimond et al. (I)*; 3,370,074 to *Dimond et al. (II)*; 3,449,413 to *Hartel et al.*; 3,503,896 to *Fishman*; 3,558,678 to *Fanning (I)*; 3,560,537 to *Eller*; 3,657,293 to *Fanning (II)*; 3,671,581 to *Keenan*; 3,717,676 to *Bechara et al.*; 3,806,529 to *Havinga et al.*; 3,864,369 *Isa et al. (I)*; 3,910,973 to *Isa et al. (II)*; and 3,957,838 to *Nishino et al.* (metallic zinc or zinc compound catalyst).

A particularly preferred known process similar to the process described in the aforementioned *Nishino et al.* reference consists of reacting an alcohol of the formula $RCH_2OH$ with a caustic of the formula $M(OH)_n$ at a reaction temperature in the presence of a catalytic amount of metallic zinc or a zinc compound to produce a final reaction mixture containing carboxylic acid salts of the formula:

$$(R\overset{O}{\overset{\|}{C}}—O)_nM\ (_n=1 \text{ or } 2)$$

wherein R is generally an alkyl substituent and M is an alkali metal or an alkaline earth metal, usually sodium. The process liberates hydrogen. The process may be accomplished under pressure or under atmospheric conditions and under varying temperature conditions.

Problems which have plagued this particular process are a tendency of the reaction mixture to foam, to form solid foams and to form a solidified reaction mixture. It is believed that the foaming is caused by the agitation of the reaction mixture and the rapid liberation of hydrogen. This foaming can be extensive and cause a shut-down of the reaction. It is also believed that the formation of solid foams and a solid reaction mixture are due to the fact that the melting point of the carboxylic acid salts produced may be higher than the reaction temperature or higher than the temperature which the reaction mixture is cooled down to for performance on the reaction mixture of a subsequent process step, e.g. acidification to carboxylic acid. It is believed that the solid foam is formed when hydrogen, which is liberated from the reaction mixture, becomes entrapped in the solidifying reaction mixture. The solid foams and solidified reaction mixture make it extremely difficult to remove the reaction mixture from the reaction zone for conveyance to a subsequent process step or the performance of a subsequent step on a reaction mixture. All the aforementioned problems are particularly prevalent in reacting alcohols of higher chain lengths, i.e. R equal to or greater than 5, but may be present when reacting lower chain length alcohols.

An attempt to solve the aforementioned problem is described in our European Patent Application of even date herewith entitled "Production of mixture of Branched and Linear Carboxylic Acid Salts"

and claiming priority from United States Application No. 106979. In this attempt the solution of such problems is sought by introducing into the reaction mixture at a specific time during the reaction an effective amount of an inert diluent for the reaction mixture.

United States Patent Number 1,926,068 to *Strosaker* describes adding an aliphatic alcohol mixture of a primary alcohol and a secondary alcohol to a fused alkaline mixture of, for example, sodium hydroxide and potassium hydroxide; U.S. Patent 2,766,267 to *Hill* describes, for example, slowly introducing into a slurry of potassium hydroxide preheated in neutral oil to a temperature between 260°C and 270°C tridecyl alcohol; and U.S. Patent 3,910,973 to *Isa et al. (II)* describes, for example, placing a solid caustic soda and titanium dioxide in an autoclave, heating the autoclave to 280°C. and then adding a mixture of $C_{12}$ and $C_{13}$ alcohols. None of these issued U.S. patents mention any of the aforementioned problems, nor are they directed to solving such problems.

Objects and summary of invention

It is an object of this invention to provide an improved process for producing carboxylic acid salts wherein foaming and solidification of the reaction mixtures are inhibited.

This invention provides for an improved process for reacting an alcohol of the formula $RCH_2OH$ with a caustic of the formula $M(OH)_n$ at a reaction temperature in the presence of a catalytic amount of metallic zinc or a zinc compound to produce a final reaction mixture containing carboxylic acid salts of the formula:

$$(R\overset{\overset{O}{\|}}{C}—O)_nM$$

wherein:

R is selected from linear alkyl and branched alkyl of from 1 to 19 carbon atoms and composed solely of carbon and hydrogen, M is selected from the group consisting of lithium, sodium, potassium, magnesium, calcium, and barium, n is a valence factor for M, being 1 where M is selected from lithium, sodium and potassium and 2 where M is selected from magnesium, calcium and barium, and liberating hydrogen. The improvement comprises:

(a) mixing

(i) the caustic,

(ii) the catalytic amount of metallic zinc or zinc compound, and

(iii) an effective amount of an inert diluent for the final reaction mixture, wherein the effective amount is sufficient to maintain fluidity of the final reaction mixture to form an intermediate reaction mixture;

(b) heating the intermediate reaction mixture to the reaction temperature; and

(c) adding the alcohol to the intermediate reaction mixture over a period of time sufficient substantially to inhibit foaming of the intermediate reaction mixture to form the final reaction mixture.

A preferred diluent is mineral oil.

The improved process permits greater control of the process, thus reducing the tendency of the reaction mixtures to foam and solidify. This results in a reaction which can proceed to completion and a final reaction mixture which can be easily removed from the reaction zone.

Detailed description of the invention

Generally, the reaction occurs at reaction temperatures of from 150°C. to 350°C. in about a 1:1 molar ratio of alcohol to (—OH) groups in the $M(OH)_n$. The carboxylic acid salts produced correspond to the carbon skeletel structure of the alcohols reacted, being of the formula $(RCOO)_nM$.

The pressures typically involved in the reaction may range from about atmospheric pressure up to pressures of the order of 69 to 103 bars (1000 to 1500 psig.) and higher, particularly where there is considerable water present, as for example, where the caustic is introduced with water. The pressure may be autogeneous. It is preferred that the process be carried out at atmospheric pressures.

It has been found that the problems indicated previously are especially prevalent when reacting an alcohol wherein R is equal to 5 or greater and in particular when R is equal to or greater than 6, and most prevalent when R is equal to or greater than 7. The process is useful for alcohols wherein R is 19 or less. Above such carbon chain lengths, it is difficult (but not impossible) to find a suitable inert diluent for the reaction mixture. Preferred ranges of R are from 5 to 15 carbon atoms, 6 to 13 carbon atoms and 7 to 11 carbon atoms with the effectiveness of the process, particularly with the inert diluent exemplified herein, respectively, increasing.

The alcohols which may be used range from substantially pure alcohols to various mixtures of alcohols such as those available in various commercial mixtures. One such commercial mixture is Monsanto's OXO ALCOHOL 7911 which generally consists of about 35% branched alcohol and about 65% linear chain alcohols and about an equal mixture of 7, 9 and 11, (i.e. R is 6, 8 and 10) carbon atoms. Table I, below, describes the properties of Monsanto's OXO ALCOHOL 7911:

TABLE I
Monsanto Oxo Alcohol 7911

| | |
|---|---|
| Composition: | |
| Linear Alcohols | 65% min. |
| Branched Alcohols | 35% max. |
| Heptanols | 30—34% |
| Nonanols | 35—43% |
| Undecanols | 27—31% |
| Alcohol content | 98.5% min. |
| Appearance | Clear liquid |
| Color, APHA | 8 max. |
| Specific gravity 20°/20°C. | 0.826—0.832 |
| Refractive index at 25°C. | 1.420—1.440 |
| Acidity (as acetic acid) | 0.03% max. |
| Aldehydes (as $C_7C_9C_{11}$) | 0.14% max. |
| Moisture | 0.1% max. |
| Boiling point range | 178—238°C. |
| Melting point | --65° to −79°C. |
| Flash point (open cup) | 190°F. |

Other similar type mixtures of branched and straight chain alcohols may also be used.

In the foregoing formula M (cation) is an alkali metal or alkaline earth metal. A preferred group consists of lithium, sodium, potassium, magnesium, calcium and barium, both individually and in various combinations, e.g. mixtures of sodium, magnesium and calcium.

In the foregoing formula for the carboxylic acid salts and in the formula for the caustic, i.e. $M(OH)_n$, n is a conventional valence factor for the metal M, being 1 for those molecules wherein M is an alkali metal such as sodium, lithium, potassium and being 2 where M is an alkaline earth metal such as calcium, magnesium and barium.

Zinc oxide is preferred as the catalyst in the process of this invention, however, excellent results may be obtained with zinc powder and other zinc compounds including, for example, zinc chloride, zinc carbonate, and zinc 2-ethylhexanoate. The catalysts are employed in a catalytic amount which may vary according to the specific reactants. Generally, however, from .2 to 3.5% by weight (calculated on the basis of Zn) based on the weight of the alcohol is employed.

The carboxylic acid salts produced by this reaction may subsequently be acidified with mineral acid to produce the corresponding carboxylic acids.

In the reaction of the foregoing alcohols and caustic to produce the corresponding carboxylic acid salts, molecular hydrogen ($H_2$) is liberated as an off-gas at substantially the precise site as the location of the hydroxyl groups in the starting alcohol molecules, the hydrogen being liberated in a ratio of 2 molecules thereof per molecule of starting alcohol $RCH_2OH$. The liberation of hydrogen continues even with the maintenance of autogenous pressure in excess of many thousands of pounds per square inch. It is believed that this forced liberation and release of hydrogen gas and other volatile materials bubbling through the reaction mixture, along with the agitation of the reaction mixture, tends to promote foaming. If the reaction mixture has a tendency to solidify, possibly, due to the production of carboxylic acid salts having a higher melting point than the reaction mixture, solid foam may be produced.

The present invention seeks to inhibit foam formation and solidification of the intermediate and final reaction mixtures by first mixing the caustic, the catalytic amount of metallic zinc or zinc compound, and an effective amount of inert diluent to form an intermediate reaction mixture, then heating the intermediate reaction mixture to the reaction temperature and then adding the alcohol to the intermediate reaction mixture over a period of time to form the final reaction mixture.

The selection of the diluent depends to a large extent on the particular alcohol reactant. It is essential that the diluent remain substantially liquid under the conditions of the reaction, i.e. have a low volatility at the temperatures and pressures of reaction. Additionally, the diluent should not polymerize and/or degrade at the temperatures and pressures of reaction. It is highly desirable that the diluent be capable of separation from the reaction mixture after the reaction is complete or after subsequent reaction steps, either by distillation or phase separation. Preferably, the diluent may be recovered by, for example, phase separation or distillation, for reuse.

The diluent is used in an effective amount of preferably from 5% to 100%, more preferably from 20% to 80%, by weight of the final reaction mixture.

A preferred diluent is a mineral oil. The mineral oils which may be employed in carrying out the instant invention are well known in the art. A preferred mineral oil for use in this invention is SOLTROL (Registered Trade Mark) 170 by Phillips Petroleum Company, Inc., Bartletsville, Oklahoma, USA. Table II, below, describes the properties of SOLTROL 170.

TABLE II
SOLTROL 170 from Phillips Chemical Company

Hydrocarbon mixture of $C_{13-14}$ Isoparaffins

| Property | Typical | Specification Minimum | Specification Maximum | Test method |
|---|---|---|---|---|
| Distillation, rec., F at 760 | | | | ASTM D 86 |
| IBP | 423(217°C) | 420 | — | |
| 10% | 431 | — | — | |
| 50% | 438 | 430 | 450 | |
| 90% | 453 | — | — | |
| EP | 468(242°C) | 450 | 475 | |
| Specific gravity 60/60F. | 0.785 | — | — | ASTM D 1298 |
| Density at 60 F, lb/gal | 6.53 | — | — | ASTM D 1250 |
| Bromine number | 1.8 | — | — | ASTM D 1159 |
| Flash point, F | 185 | 175 | — | ASTM D 56 |
| Saybolt color | 30 | 25 | — | ASTM D 156 |
| Sulfur content, wt. percent | 0.0010 | — | — | ASTM D 1266 |
| Acidity of Distillation Residue | Neutral | Neutral | Neutral | ASTM D 1093 |
| Aniline point, F. | 192 | 190 | — | ASTM D 1012 |
| Copper corrosion, 3 hrs. at 212 F. | 1 | — | 1 | ASTM D 130 |
| Kauri-butanol value | 24.6 | — | — | ASTM D 1133 |
| Kinematic viscosity, cs at 100 f. | 2.51 | — | — | ASTM D 445 |

The addition of the alcohol to the intermediate reaction mixture takes place over a period of time. This period of time should be sufficient to permit control of the process to substantially inhibit foaming of the intermediate reaction mixture. In effect, the alcohol is added to the intermediate reaction mixture so that there is a comparatively slow liberation of hydrogen. In prior art processes when the caustic, metallic zinc or zinc compound catalyst and alcohol were all mixed in one reaction zone and heated, there was a tremendous on-rush of hydrogen bubbling through the reaction mixture. The slow addition of alcohol tends to control this liberation of hydrogen and thus control foaming. The time in which the alcohol may be added to the intermediate reaction mixture may be readily determined by one skilled in the art by viewing the reaction zone and foaming present therein. Typically, for example, the addition may occur over a period of from 1/2 hour to 4 hours, although under certain conditions, depending on the quantity of diluent present, the particular alcohols reacted and the temperature of the reaction, the time may be greater or less.

The process of this invention has been found particularly useful in inhibiting foaming and solidification of a reaction mixture wherein the alcohol reacted is, for example, Monsanto's 7911 alcohol.

This invention, in effect, combines the inhibition of foaming caused by the inert diluent and its decrease of the viscosity of the reaction mixtures, and the inhibition of foaming caused by the slow addition of the alcohol to the intermediate reaction mixture. The inert diluent additionally inhibits the solidification of the reaction mixtures. The mechanism by which the instant process achieves the desired results is not completely understood. In summary, however, it is believed, that the diluent employed herein decreases the viscosity of the reaction mixture to allow for the escape of hydrogen therefrom in such a manner as to reduce foaming caused by such hydrogen liberation and to reduce the quantity of hydrogen entrapped in the reaction mixture. It is believed that the controlled addition of the

alcohol to the intermediate reaction mixture decreases the quantity of hydrogen liberated over a period of time so that there is not an on rush of bubbling of hydrogen through the intermediate reaction mixture. It is also believed that the diluent dissolves the reaction mixtures to prevent solidification thereof.

The following examples are illustrative of the invention and are not to be regarded as limitative.

Comparative Example I
No Diluent-Alcohol introduced at beginning of reaction

Equipment
Reaction vessel

A one-liter three-neck, glass round-bottom flask was used as the reaction vessel. In the center neck a simple glass paddle design blade with glass rod stirrer was connected to a variable high speed stirrer motor. On another neck a "Y" shaped adapter was placed to allow for a thermometer for measuring the reaction mixture temperature and an addition funnel containing water. The addition funnel had a side arm with stopcock. The addition funnel was also equipped with nitrogen inlet for purging both the funnel and the reaction vessel.

On the other neck was placed another "Y" shaped adapter. On this adapter was fitted a thermometer to measure vapor temperatures and a Dean Stark trap with condenser. The Dean Stark trap had provisions for draining bottom layers (water in this case) and also for recycle of the lower portion into the reaction flask (total reflux).

The condenser outlet on the Dean Stark trap led to a container to catch any foam or liquids uncondensed by the condenser. The foam container was connected by a tee to a positive nitrogen source and a Precision Scientific Wet test meter. The meter gave both total gas evolution and instantaneous gas evolution rates. The wet test flowmeter also served to isolate the system from air and prevent flashbacks in the event of hydrogen ignition. The exit port from the wet test meter was to an exhaust hood.

Heat bath

Oil was used as the heat transfer agent. A 600 watt thermowatch heat source for the oil was supplemented when necessary with a hot plate type heater-stirrer. A high grade silicon oil with a flash point of 315°C was the heat transfer agent in the bath.

Reactants

Monsanto Oxo Alcohol 7911 alcohol
Technical grade sodium hydroxide flakes
Reagent grade zinc oxide powder
Reagent grade calcium hydroxide powder
Magnesium hydroxide powder

Comparative process

Into the reaction vessel was placed 388.8 g of Monsanto's 7911 alcohol (2.69 mole). While stirring, 122.4 g of sodium hydroxide (2.97 mole), 3.9 g of zinc oxide, 5.9 g of calcium hydroxide and 5.9 g of magnesium hydroxide were then added to the vessel. The vessel was sealed and purged with nitrogen and water was placed in the addition funnel and heating commenced. After initial water removal to initiate reaction, an attempt was made to control the reaction by adding water back from the addition funnel. Table III shows the course of the reaction with time zero being when vapors first distilled. At 25 minutes it was noted that the flask was 63% full and at 40 minutes the onset of foaming occurred. The flask was 75% full. Increasing stirring speed helped reduce the foam somewhat, but not completely. At 47 minutes the flask was almost completely filled with foam. Addition of water was commenced in an attempt to control the foam. At 51 minutes the foam had gone down some but the flask was still 88% full. At 61 minutes the flask was again filled with foam. At 83 minutes the foam appeared to subside but reappeared at 96 minutes. A few minutes later, the foam increased to fill the entire apparatus. The foam solidified in cooler parts of the apparatus blocking the escape of hydrogen. At this point the clamped ball joints of the reactor connected to the rest of the apparatus separated. Hydrogen and foam then came out of the separated ball joints. Since approximately 120 liters of hydrogen would be liberated by the reaction at standard temperature and pressure, we had completed approximately 67% of reaction (81 liters). The reaction was terminated.

TABLE III

| Time (min) | Oil temp. (°C) | Reactor temp. (°C) | Vapor temp. (°C) | Total $H_2O$ Out (Ml.) | Total $H_2O$ In (Ml.) | $H_2$ rate (l/hr) | $H_2$ total (l) |
|---|---|---|---|---|---|---|---|
| 0 | 237 | 182 | 97 | 0 | 0 | 0 | 0 |
| 7 | 240 | 186 | 154 | 6.0 | 0 | 4 | 0.8 |
| 15 | 249 | 189 | 156 | 9.2 | 0 | 4 | 1.5 |
| 25 | 253 | 192 | 154 | 12.6 | 0 | 22 | 4.8 |
| 40 | 258 | 192 | 135 | 15.2 | 0 | 34 | 14.0 |
| 47 | 261 | 190 | 136 | 15.6 | 0 | 52 | 18.9 |
| 51 | 265 | 189 | 133 | 15.6 | 0 | 36 | 22.8 |
| 57 | 267 | 191 | 132 | 15.6 | 3.0 | 36 | 26.0 |
| 61 | 259 | 191 | 133 | 15.6 | 3.0 | — | 28.0 |
| 67 | 249 | 192 | — | 15.6 | 3.0 | 48 | 32.0 |
| 83 | 241 | 189 | 125 | 15.6 | 14.6 | 63 | 45 |
| 96 | 241 | 190 | 122 | 15.6 | 14.6 | 87 | 61.5 |
| — | — | — | — | — | — | — | 81 |

Comparative Example II

Diluent and alcohol introduced at beginning of reaction

The equipment described in Comparative Example I was charged with Monsanto's 7911 alcohol (260 g) and Soltrol 170 (96 g). Sodium hydroxide flake (81 g), magnesium hydroxide (3.9 g), calcium hydroxide (3.9 g) and zinc oxide (2.6 g) were added to the stirring mixture. The reactor was sealed and stirring maintained at 200 RPM. Heat was applied. When the mixture reached a temperature of 194°C distillate began collecting in the phase separator. The organic layer was returned to the reaction mixture while the water was collected. After 8.0 ml of water collected, the hydrogen evolution rate had risen from 0 to 320 cc/min. and the reaction temperature was now 202°C. Small increments of water were added back to the mixture over the next 3 hours in order to maintain the hydrogen evolution rate at 300—800 cc/min. The total amount of water added was 8.0 ml. Only 85% of the theoretical amount of hydrogen evolved.

Very little foaming occurred, but it was present. A small portion (10 g) of soap mixture was withdrawn from the reactor at this point and acidified carefully with 25 ml of concentrate a hydrochloric acid. The organic phase was separated, dried over magnesium sulfate, anhydrous, and analyzed by gas chromatography. The analysis mixture contained 24% branched acid.

After an additional 6 hours of heating the reaction mixture was reanalyzed as above and found to still contain 24% branched acid.

The Monsanto 7911 alcohol used had analyzed composition of 30% branched and 70% linear alcohol.

Example 1

To a 500 cc. three-necked reactor fitted with a reflux condensor, stirrer, and addition funnel is charged sodium hydroxide (50 g), SOLTROL 170 (65 g), zinc oxide (2.0 g), magnesium hydroxide (2.0 g) and calcium hydroxide (2.0 g). The mixture was heated to 210°C. Monsanto 7911 alcohol (45 ml.) was added over the next four hours while 10.24 liters of hydrogen evolved. No foaming or solidification occurred. The crude product was removed by water wash. The water layer was acidified.

Gas chromatograph analysis indicated that only linear acids were produced.

## Claims

1. A process for reacting an alcohol of the formula $RCH_2OH$ with a caustic of the formula $M(OH)_n$ at a reaction temperature in the presence of a catalytic amount of metallic zinc or a zinc compound to produce a final reaction mixture containing carboxylic acid salts of the formula:

$$(R\overset{\overset{O}{\|}}{C}—O)_nM$$

wherein:

R is selected from linear alkyl and branched alkyl of from 1 to 19 carbon atoms and composed solely of carbon and hydrogen;

M is selected from the group consisting of lithium, sodium, potassium, magnesium, calcium, and barium;

n is a valence factor for M, being 1 where M is selected from lithium, sodium and potassium and 2

where M is selected from magnesium, calcium, and barium, and liberating hydrogen, characterised by the following steps:—

(a) mixing

(i) the caustic,

(ii) the catalytic amount of metallic zinc or zinc compound, and

(iii) an effective amount of an inert diluent for the final reaction mixture, wherein the effective amount is sufficient to maintain fluidity of the final reaction mixture to form an intermediate reaction mixture;

(b) heating the intermediate reaction mixture to the reaction temperature; and

(c) adding the alcohol to the intermediate reaction mixture over a period of time sufficient substantially to inhibit foaming of the intermediate reaction mixture to form the final reaction mixture.

2. A process as claimed in Claim 1, characterised in that R has from 5 to 15 carbon atoms.

3. A process as claimed in Claim 1, characterised in that R has from 6 to 13 carbon atoms.

4. A process as claimed in Claim 1, characterised in that R has from 7 to 11 carbon atoms.

5. A process as claimed in any of Claims 1 to 4 characterised in that the caustic is sodium hydroxide.

6. A process as claimed in any of Claims 1 to 5 characterised in that the diluent is a mineral oil.

7. A process as claimed in any of Claims 1 to 6 characterised in that the effective amount of diluent is from 5% to 100% by weight of the final reaction mixture.

8. A process as claimed in Claim 7 characterised in that the effective amount of diluent is from 20% to 80% by weight of the final reaction mixture.

9. A process as claimed in any of Claims 1 to 8 characterised in that the catalyst is zinc oxide.

**Revendications**

1. Un procédé de réaction d'un alcool de formule $RCH_2OH$ avec un base caustique de formule $M(OH)_n$ à une température réactionnelle en présence d'une quantité catalytique de zinc métallique ou d'un composé à base de zinc pour produire un mélange réactionnel final contenant des sels d'acides carboxyliques de formule:

$$\left(R\overset{\overset{O}{\|}}{C}-O\right)_n M$$

dans laquelle:

R est choisi parmi les radicaux alkyles linéaires et ramifiés contenant de 1 à 19 atomes de carbone et exclusivement formés de carbone et d'hydrogène;

M est choisi dans le groupe comprenant lithium, sodium, potassium, magnésium, calcium et baryum;

n est le facteur de valence de M, n étant égal à 1 étant M consiste en lithium, sodium et potassium, et égal à 2 lorsque M consiste en magnésium, calcium et baryum, et libérant l'hydrogène, caractérisé par les étapes suivantes:

a) mélange

(i) de la base caustique,

(ii) de la quantité catalytique de zinc métallique ou de composé à base de zinc, et

(iii) une quantité efficace d'un diluant inerte vis-à-vis du mélange réactionnel final dans lequel la quantité efficace est suffisante pour maintenir fluide le mélange réactionnel final, afin de former un mélange réactionnel intermédiaire;

b) chauffage du mélange réactionnel intermédiaire à la température réactionnelle; et,

c) addition de l'alcool au mélange réactionnel intermédiaire sur une période de temps suffisante pour pratiquement inhiber tout moussage du mélange réactionnel intermédiaire et former le mélange réactionnel final.

2. Un procédé tel que revendiqué dans la revendication 1, caractérisé en ce que R comprend de 5 à 15 atomes de carbone.

3. Un procédé tel que revendiqué dans la revendication 1, caractérisé en ce que R comprend de 6 à 13 atomes de carbone.

4. Un procédé tel que revendiqué dans la revendication 1, caractérisé en ce que R comprend de 7 à 11 atomes de carbone.

5. Un procédé tel que revendiqué dans l'une quelconque des revendications 1 à 4, caractérisé en ce que la base caustique est de l'hydroxyde de sodium.

6. Un procédé tel que revendiqué dans l'une quelconque des revendications 1 à 5, caractérisé en que le diluant est une huile minérale.

7. Un procédé tel que revendiqué dans l'une quelconque des revendications 1 à 6, caractérisé en ce que la quantité efficace de diluant est comprise entre 5 et 100% en poids du mélange réactionnel final.

8. Un procédé tel que revendiqué dans la revendication 7, caractérisé en ce que la quantité efficace de diluant forme de 20 à 80% en poids du mélange réactionnel final.

9. Un procédé tel que revendiqué dans l'une quelconque des revendications 1 à 8, caractérisé en ce que le catalyseur est de l'oxyde de zinc.

**Patentansprüche**

1. Verfahren zur Reaktion eines Alkohols der Formel $RCH_2OH$ mit einer Base der Formel $M(OH)_n$ bei einer Reaktionstemperatur in Gegenwart einer katalytischen Menge an metallischem Zink oder einer Zinkverbindung, um eine Endreaktionsmischung zu erzeugen, die Karbonsäuresalze der Formel

$$(R\overset{\overset{O}{\|}}{C}—O)_nM$$

enthält, wobei

R aus linearem Alkyl oder verzweigtem Alkyl mit von 1 bis 19 C-Atomen gewählt wird und nur aus Kohlenstoff und Wasserstoff besteht,

M aus der Gruppe gewählt wird, die aus Lithium, Natrium, Kalium, Magnesium, Calcium and Barium besteht,

n ein Valenzfaktor für M ist, der 1 ist, wenn M aus Lithium, Natrium and Kalium gewählt wird, und 2 ist, wenn M aus Magnesium, Calcium und Barium gewählt wird, und Wasserstoff freigesetzt wird, gekennzeichnet durch die folgenden Schritte:

a) Mischen

1) der Base,

2) der katalytischen Menge an metallischem Zink oder der Zink-verbindung und

3) einer wirksamen Menge eines inerten Verdünnungsmittels für die Endreaktionsmischung, bei der die wirksame Menge ausreicht, den flüssigen Zustand der Endreaktionsmischung zu erhalten, um eine Zwischenreaktionsmischung zu bilden,

b) Erwärmen der Zwischenreaktionsmischung auf die Reaktionstemperatur und

c) Zusetzen des Alkohols zu der Zwischenreaktionsmischung über einen Zeitraum, der ausreicht, im wesentlichen das Schäumen der Zwischenreaktionsmischung zu verhindern, um die Endreaktionsmischung zu bilden.

2. Verfahren nach Anspruch 1, dadurch gekennzeichnet, daß R von 5 bis 15 C-Atome hat.

3. Verfahren nach Anspruch 1, dadurch gekennzeichnet, daß R von 6 bis 13 C-Atome hat.

4. Verfahren nach Anspruch 1, dadurch gekennzeichnet, daß R von 7 bis 11 C-Atome hat.

5. Verfahren nach einem der Ansprüche 1 bis 4, dadurch gekennzeichnet, daß die Base Natriumhydroxid ist.

6. Verfahren nach einem der Ansprüche 1 bis 5, dadurch gekennzeichnet, daß das Verdünnungsmittel ein Mineralöl ist.

7. Verfahren nach einem der Ansprüche 1 bis 6, dadurch gekennzeichnet, daß die wirksame Menge des Verdünnungsmittels von 5 bis 100 Gew.% der Endreaktionsmischung ist.

8. Verfahren nach Anspruch 7, dadurch gekennzeichnet, daß die wirksame Menge des Verdünnungsmittels von 20 bis 80 Gew.-% der Endreaktionsmischung ist.

9. Verfahren nach einem der Ansprüche 1 bis 8, dadurch gekennzeichnet, daß der Katalysator Zinkoxid ist.